# EUROPEAN PATENT SPECIFICATION

(11) **EP 0 887 098 B1**
(45) Date of publication and mention of the grant of the patent: **14.06.2006**
(21) Application number: 98114859.6
(22) Date of filing: 10.09.1992
(51) Int. Cl.: B01D 39/16, B01D 67/00, B29C 59/14, A61M 1/34, D06M 10/02, A61M 1/36, B01D 53/22

(54) **Gas plasma treated porous medium and method of separation using same**
Gasplasma behandeltes, poröses Medium und Trennungsmethode unter Verwendung des Mediums
Milieu poreux traité au plasma gazeux et procédé de séparation utilisant un tel milieu

(30) Priority: 11.09.1991 US 756567; 11.09.1991 US 757535
(43) Date of publication of application: 30.12.1998
(62) Divisional of application: 92921260.3
(73) Proprietor: PALL CORPORATION, East Hills, New York 11548 (US)
(72) Inventor: Gsell, Thomas C., Glen Cove, NY 11452 (US)
(74) Representative: Hartley, Andrew Philip

(56) References cited:
- EP-A- 0 134 055
- EP-A- 0 177 777
- DE-A- 3 712 491
- US-A- 4 199 448
- US-A- 4 806 246
- US-A- 4 824 444
- US-A- 4 933 092
- PATENT ABSTRACTS OF JAPAN vol. 014, no. 014 (C-674), 12 January 1990 (1990-01-12) & JP 01 256971 A (KURARAY CO LTD), 13 October 1989 (1989-10-13) -& DATABASE WPI Section Ch, Week 8947 Derwent Publications Ltd., London, GB; Class A17, AN 89-344405 XP002115917 & JP 01 256971 A (KURARAY CO. LTD.), 12 January 1990 (1990-01-12)
- PATENT ABSTRACTS OF JAPAN vol. 005, no. 008 (C-039), 20 January 1981 (1981-01-20) & JP 55 137138 A (TORAY IND INC), 25 October 1980 (1980-10-25)

## Description

### Technical Field

This invention relates to a gas plasma treated porous medium and the use of such a porous medium to separate or remove components from biological fluids such as blood.

### Background of the Invention

Many substances, particularly fluids, are passed through porous media to separate or remove certain materials. In particular, biological fluids, containing a variety of components and constituents, are typically passed through porous media for such purposes. For example, it is sometimes desirable to separate whole blood into one or more component parts and/or to separate a constituent such as leukocytes and/or complement from whole blood or a blood component. Thus, improved porous media and separation techniques are desirable to separate and/or remove a component or a constituent from a fluid.

The depletion of undesirable matter from biological fluids administered to a patient for therapeutic reasons is of considerable importance to avoid potential harmful effects on the recipient of the fluid. Of particular importance is the depletion of deleterious matter, especially leukocytes, from biological fluids such as blood and blood products used in transfusions and in extracorporeal circuits to prevent or reduce reperfusion injury, as well as a number of other diseases and conditions.

For example, with respect to biological fluids used in transfusions, in the currently used centrifugal methods for separating blood into components, leukocytes may be present in the packed red cell, plasma, and platelet-containing fractions. It is desirable to reduce the leukocyte concentration of these blood components to as low a level as possible. While there is no firm criterion, it is accepted that many of the undesirable effects of transfusion would be reduced if the leukocyte content were reduced by a factor of about 100 or more prior to administration. It may also be desirable to reduce the complement concentration, more particularly that of biologically active complement fragments, e.g., C3a, to a low level.

With respect to biological fluids used in extracorporeal circuits (e.g., the extracorporeal circuit for heart bypass operations), since current methods may lead to leukocyte activation, if leukocytes are activated but lack an appropriate antigenic target, these methods may lead to leukocyte-inflicted damage to internal organs, particularly ischemic tissues, i.e., tissues in which no blood is flowing such as the heart and lungs during certain surgical procedures.

Moreover, with increasing frequency, the most common leukocyte, the granulocytic neutrophil, has been implicated as the mediator of tissue destructive events in a variety of disorders, including reperfusion injury, respiratory distress syndromes, rheumatoid arthritis, skin disorders, and ulcerative colitis. The commonality which pervades these pathologies is the neutrophil's ability to release a number of agents which can disrupt and destroy normal cellular function, dissolve connective tissue, and cause injury to organs.

It has also been shown that circulating leukocytes contribute to or mediate ischemic and reperfusion injury during organ preservation, particularly following extended preservation of the heart-lung bloc commonly required during cardiopulmonary bypass operations (CPB). Leukocytes have also been associated with increased oxygen radical activity, pulmonary edema, and vasoconstriction.

JP-A-01256971 is directed to a plasma treated nonwoven fabric for use as a leukocyte removing filter. The filter comprises a container having an inlet and an outlet for solutions containing leukocytes that is filled with the nonwoven fabric.

JP-A-55137138 is directed to a polyvinyl chloride resin molded product that is treated with low-temperature plasma of a gas mixture of CO or Ar with N₂ + H₂ or NH₃. A crosslinked layer which prevents exudation of additives and a modified surface having hydrophilicity and blood-compatibility is obtained.

DE 37 12 491 A is directed to hydrophobic microporous microfiltration membranes having a permanent hydrophilic surface and a process for preparing these membranes. The surface of a hydrophobic microporous microfiltration membrane substrate is treated with a non-polymerizable plasma gas.

### Summary of the Invention

In accordance with a first aspect of the invention, there is provided a leukocyte depletion medium for removing leukocytes from a biological fluid comprising: a fibrous porous medium which has been treated with a plasma generated from a mixture of gases, the mixture of gases including argon.

In accordance with a second aspect of the invention, there is provided a method for the separation of leukocytes from a biological fluid comprising: passing the fluid through a leukocyte depletion medium comprising a fibrous porous medium which has been treated with a gas plasma generated from a mixture of gases including argon, and depleting leukocytes from the biological fluid.

In accordance with a third aspect of the invention, there is provided a method for making a leukocyte depletion medium comprising: generating a plasma from a mixture of gases including argon; and treating a fibrous porous medium with the plasma.

Additionally, a porous medium produced by the present invention minimizes platelet loss from a platelet-containing biological fluid passing therethrough. Moreover, a porous medium produced by the present invention minimizes the loss of desirable components of the biological fluid such as Factor VIII. Further, a porous medium produced in accordance with the present invention minimizes the affect on desirable characteristics of the biological fluid contacting the porous medium, e.g., the clotting time.

The porous medium provided by the present invention is advantageous for several reasons. The leukocyte depletion capability of the gas plasma treated porous medium is improved over that of non-gas plasma treated media, as is the rate of leukocyte removal compared to that of using non-gas plasma treated media. Further, the ability of the porous medium to pass platelets therethrough is enhanced since the gas plasma treated fibers have a relatively low adhesiveness to platelets. As a result, a larger proportion of the platelets pass through the porous medium, a characteristic which is highly beneficial, particularly during surgery, collecting and processing donated blood, and other similar protocols.

Among the additional advantages, the products provided by the present invention have very low amounts of leachable matter that could contaminate the liquid being filtered. Additionally, it is significantly more efficient to prepare the fibers by using the present invention than it is to prepare fibers using other surface modification protocols since the present invention does not use organic reagents in preparing the fibers modified by gas plasma and there is no need to wash or dry the fibers to remove extractables prior to use.

The present invention provides a gas plasma treated porous medium which may be included in a filter assembly for removing leukocytes and/or other undesirable matter from a liquid, such as blood. A filter assembly as provided by the present invention generally comprises a housing and a porous medium. The housing has an inlet and an outlet and defines a liquid flow path between the inlet and the outlet. The gas plasma treated porous medium is positioned inside the housing across the liquid flow path.

The present invention relates to any fibrous porous medium which has been treated with a plasma generated from a mixture of gases including argon and its use with respect to any protocol involving processing of fluid, particularly biological fluid. Interspersed in the following specification, reference will be made to an embodiment relating to extracorporeal circulation. The invention is not intended to be limited thereby.

In an extracorporeal embodiment of the filter assembly, the gas plasma treated medium includes a fibrous structure for decreasing the leukocyte content of the liquid, typically at a flow rate greater than about 25 milliliters per minute, more typically at a flow rate greater than about 150 milliliters per minute.

Filter assemblies provided by the present invention include a fibrous porous medium which has been treated with a plasma generated from a mixture of gases including argon. In some embodiments, e.g., in an extracorporeal circuit, the filter assembly may also have one or more of the following characteristics: a hollow, generally cylindrical configuration; a total fibrous surface area greater than about 1.5 square meters; and a critical wetting surface tension (CWST) of 53 dynes per centimeter or more. The filter assemblies may have a pleated structure, a total hold-up volume up to about 400 cubic centimeters, and may further comprise a porous degassing element for removing gas from the liquid, a liquophobic membrane which allows gas but not liquid to escape from the housing, and/or a vent for removing gas from the housing.

With respect to an extracorporeal embodiment, a method provided by the present invention may comprise decreasing the leukocyte content of the liquid by passing the liquid through a gas plasma treated fibrous medium having one or more of the characteristics noted above.

Filter assemblies and methods provided by the present invention are particularly advantageous. They remove leukocytes and leukocyte-type cells very effectively. In an embodiment involving a fibrous medium, leukocytes are not only trapped in the interstices of the fibrous medium, but they also adhere to the surfaces of the fibers in the medium. The fibrous medium provides ample surface area on which the leukocytes can adhere. Furthermore, the gas plasma treated fibrous medium allows the biological fluid to readily wet the fibrous medium, actively seep into all of the interstices of the medium, and completely contact the ample surface area of the fibers.

Filter assemblies and methods embodying an extracorporeal embodiment provided by the present invention are capable of removing leukocytes while maintaining a large flow of liquid through the fibrous medium for a considerable span of time without clogging or plugging. Conventional filters may remove leukocytes at low flows, e.g., 5-10 milliliters per minute, but embodiments provided by the present invention are capable of removing leukocytes at much greater flow rates, even hundreds of times greater. Also, the fibrous medium, treated as noted below, not only permits the passage of a greater proportion of platelets than heretofore possible, but also selectively increases the percentage of neutrophils removed from the liquid. As noted previously, the liquid flows through the medium with minimal resistance and resists plugging or clogging. Thus, although embodiments provided by the present invention are nonetheless effective at low flow rates, they are capable of removing leukocytes at very large flow rates for extended periods of time. For example, leukocytes may be removed from a liquid such as blood at a flow rate up to six liters per minute for three to four hours and, in some cases up to ten hours, without clogging or plugging.

A filter assembly or method provided by the present invention may be used in an extracorporeal circuit, and/or may be employed for therapeutic applications, including but not limited to cardiopulmonary bypass operations or the like, autologous transfusion, leucopheresis, apheresis, or dialysis. Thus, the device and method have application whenever a biological fluid, such as blood or a leukocyte-containing liquid, is brought into contact with external circuitry, and thence returned to the body or specific organs.

A filter assembly or method provided by the present invention may also be used for a number of other therapeutic or surgical protocols, including, but not limited to cardioplegia or coronary perfusion, in order to perfuse and maintain safe levels of metabolic activity within tissues and organs; for myocardial infarcted patients to reduce subsequent damage during reperfusion in the affected heart region; and to reduce or eliminate the deleterious effects attributed to a wide variety of injuries, diseases, or conditions. Passing the patient's blood through a device provided by the present invention can be used in clinical or therapeutic regimens in which leukocyte depletion is beneficial. Also, the media provided by the present invention can be used clinically or therapeutically to remove cancer cells and the like which are structurally similar to leukocytes.

### Brief Description of the Drawings

Figure 1 is a cross section of a typical extracorporeal filter assembly having a pleated porous medium positioned inside a housing.
Figure 2 is a top view of the filter assembly of Figure 1, including the cover and a top portion of the body of the housing.

### Detailed Description of the Preferred Embodiments

The present invention relates to a porous medium for separating or removing substances from fluids comprising a fibrous porous medium which has been treated with a plasma generated from a mixture of gases including argon. The present invention also concerns a method of using such a gas plasma treated porous medium to separate or remove materials from fluids. In particular, the present invention provides for a porous medium and method for the separation or depletion of undesirable matter such as leukocytes and/or complement from a biological fluid such as blood or blood products. Further, the present invention provides a porous medium for desirable material such as platelets or coagulation factors, e.g., Factor VIII, to pass therethrough. The present invention also provides for the efficient removal of complement, e.g., C3a. The porous medium provides for passage of the biological fluid without significantly affecting desirable characteristics of the fluid, e.g., the clotting time.

The present invention may also be useful while collecting and processing biological fluid, e.g., while separating blood into components, or in therapeutic applications such as repeatedly recirculating whole blood through a porous medium, e.g., in an extracorporeal circuit.

As provided by the present invention, any fibrous porous medium can be treated with a plasma generated from a mixture of gases including argon. The porous medium provided by this invention may be formed by any suitable fibrous material which will be modified by treatment with gas plasma. The present inventive porous medium may be formed of natural or synthetic fibers.

Synthetic resins from which fibers are prepared commercially and which are suitable for use in the subject invention include, but are not limited to thermoplastics such as polyolefins, such as polyethylexze, polypropylene and polymethylpentene; polyamides, such as nylon 6, nylon 610, nylon 10, nylon 11, nylon 12 and nylon 66; polyesters, such as polybutylene terephthalate (PBT) and polyethylene terephthalate (PET); polysulfones; polyaramides; acrylics; polyarylene oxides and sulfides; polymers and copolymers made from halogenated olefins, such as polyvinylfluoride and polyvinylidene fluoride; polymers and copolymers made from unsaturated nitriles, such as polyacrylonitriles; and cellulose acetate. Preferred polymers are polyolefins, polyesters, and polyamides. The most preferred polymer is polybutylene terephthalate (PBT).

The present invention provides a porous medium which is treated with a gas plasma, typically a low temperature gas plasma, with or without deposition of a polymeric substance formed by the plasma or introduced into the plasma. The gas plasma is generated from a mixture of gases including argon. The porous medium may be treated with the gas plasma at any suitable point in its manufacture. For example, the porous medium may be treated with gas plasma after it has been formed into its desired shape, or the fibers which are used to make the porous medium may be treated with gas plasma prior to formation of the porous medium. It is likewise possible to treat with gas plasma a precursor to the final form of the porous medium, as, for example, where the porous medium is first formed into a sheet-like structure, and the sheet-like structure is thereafter pleated or the like to form the final configuration of the porous medium as it will be used in the device. It is preferred to treat the fiber surfaces with the gas plasma prior to formation of the porous medium.

Exemplary porous media suitable for a gas plasma treatment include the types of fibrous porous media disclosed in U.S. Patents 4,925,572; 4,880,548; 5,100,564; and International Publication Numbers WO 91/04088; and WO 92/07656.

The term "plasma" or "gas plasma" is used generally to describe the state of an ionized gas. The use of the term "plasma" in this context should not be confused with "plasma" as it refers to a biological fluid. A gas plasma consists of high energy charged ions (positive or negative), electrons, and neutral species. As known in the art, a plasma may be generated by combustion, flames, physical shock, or, preferably, by electrical discharge, such as a corona or glow discharge. It is intended that the present invention not be limited by the method of generating the plasma. In one exemplary technique, radio frequency (RF) discharge, a substrate to be treated is placed in a vacuum chamber and the chamber is evacuated. Gas at low pressure is bled into the system through the gas inbleed until the desired gas pressure differential across the conduit is achieved. An electromagnetic field is generated by subjecting the gas to a capacitive or inductive RF electrical discharge. The gas absorbs energy from the electromagnetic field and ionizes, producing high energy particles. The gas plasma, as used in the context of the present invention, is exposed to the fibers or the fibrous porous medium, thereby modifying the properties of the fibers or the porous medium to provide it with characteristics not possessed by the untreated fibers or porous medium, e.g., improving its biocompatibility, and ability to selectively remove various cellular, particulate, and dissolved material.

The fibers or the fibrous medium are treated with a plasma formed from a mixture of gases including argon.

Exemplary inorganic gases which may be used in the mixture include helium, nitrogen, neon, nitrous oxide, nitrogen dioxide, oxygen, air, ammonia, carbon monoxide, carbon dioxide, hydrogen, chlorine, hydrogen chloride, bromine cyanide, sulfur dioxide, hydrogen sulfide, xenon, krypton, and the like. Exemplary organic gases which may be used in the mixture include acetylene, pyridine, gases of organosilane compounds and organopolysiloxane compounds, fluorocarbon compounds and the like. In addition, the gas may be a vaporized organic material, such as an ethylenic monomer to be plasma polymerized or deposited on the surface of the fiber.

Typical parameters for treatment with a gas plasma may include power levels from about 10 to about 3000 watts, preferably about 500 to about 2500 watts, and most preferably about 1500 to about 2500 watts. The RF frequency may include about 1 kHz to about 100 MHz, preferably about 15 kHz to about 60 MHz, most preferably about 30 kHz to about 50 kHz. Exposure times may include about 5 seconds to about 12 hours, preferably about 1 minute to about 2 hours, most preferably about 10 to about 30 minutes. The gas pressures may include about 0.001 to 100 torr, preferably about 0.01 to 1 torr, and most preferably about 0.1 to about 0.5 torr; and a gas flow rate of about 1-2000 standard cc/min.

The gas plasma treated porous medium provided by the present invention is useful in the separation and removal of materials in any type of gaseous or liquid fluid amenable to separation or removal techniques by passage through a porous medium. The present inventive porous medium is particularly useful for the separation and removal of substances in liquid fluids, especially biological fluids. Biological fluids include any treated or untreated fluid associated with living organisms, particularly blood, including whole blood, warm or cold blood, and stored or fresh blood; treated blood, such as blood diluted with a physiological solution, including but not limited to saline, nutrient, and/or anticoagulant solutions; one or more blood components, such as platelet concentrate (PC), platelet-rich plasma (PRP), platelet-free plasma, platelet-poor plasma (PPP), plasma, packed red cells (PRC), or buffy coat (BC); and analogous blood products derived from blood or a blood component or derived from bone marrow. The biological fluid may include leukocytes, or may be treated to remove leukocytes. As used herein, blood product or biological fluid refers to the components described above, and to similar blood products or biological fluids obtained by other means and with similar properties.

The gas plasma treated porous medium provided by the present invention is particularly useful in the separation or removal of undesirable material from biological fluids. It is intended that the present invention not be limited by the type of undesirable material removed or separated. Illustrative undesirable material which may be separated from such biological fluids include deleterious matter such as activated and non-activated leukocytes (including neutrophils or granulocytic neutrophils), complement (including biological active fragments, e.g., C3a), fat emboli, microaggregates; lipids, cells which are morphologically similar to leukocytes, cellular components and material, and other debris. Other materials which may be separated from biological fluids include gas or air, cancer cells, stem cells, and the like. In one aspect of the invention, the passage of red cells through the medium may be prevented. For example, a non red cell-containing fluid may pass through a porous medium of the instant invention until the porous medium is blocked, without passing red cells therethrough.

The present inventive porous medium is particularly useful in the removal of deleterious matter, especially leukocytes, from biological fluids, such as blood and blood products. In addition, and unexpectedly, the gas plasma treated porous medium is capable of removing leukocytes from blood and blood products while allowing a substantial quantity of the platelets therein to pass therethrough. Thus, the gas plasma treated medium minimizes the loss of platelets. For example, the porous media provided by the present invention are capable of passing at least about 30% of the platelets, typically at least about 50% or more of the platelets. The present invention also provides for a porous medium which is capable of removing complement without significantly removing Factor VIII or without significantly affecting clotting time.

While the precise mechanism by which the gas plasma improves the characteristics of the porous medium to make it more suitable for the separation and removal of substances from fluids, particularly biological fluids, is not presently known, it is believed that the gas plasma modifies the surface characteristics of the porous medium, thereby affecting the interaction between the porous medium and the components of the fluid, e.g., platelets and leukocytes suspended in biological fluids. The exact nature of the surface modification is not fully understood at present. It has been observed, however, quite surprisingly and unexpectedly, that a porous medium made from fibers treated with gas plasma exhibits enhanced leukocyte depletion of blood and blood products and, beneficially, inhibited retention of platelets.

Factors which may affect the efficiency of the porous medium to separate or remove substances from fluids, particularly to deplete deleterious matter from biological fluids, and its utility for a given application, include, but are not limited to, the diameter of the fibers used to prepare the porous medium, the pore rating of the porous medium, the flow rate of the biological fluid through the porous medium, the flow area of the porous medium, the density of the fibers used to prepare the porous medium, the weight of the fibers, and the voids volume of the porous medium. The various factors can be varied singly or in combination to provide a gas plasma treated porous medium that will be suitable for the particularly desired purpose.

For example, the efficiency of leukocyte removal will generally be enhanced by increasing the weight of the fiber and reducing the voids volume of the porous medium. Care, however, needs to be taken not to use a fiber diameter so small that the porous medium will collapse at normal working differential pressure. Similarly, the flow rate may be increased by increasing the area of the porous medium with a concomitant reduction in the thickness of the medium, or by increasing the voids volume of the porous medium. Typical flow rates of about 1 cc/min to about 10,000 cc/min. A typical flow area may be in the range of from about 9.3 x 10⁻³dm² to about 93 dm² (about 001 to about 10 ft²) . A typical voids volume may be in the range from about 55% to about 95%.

While synthetic fibers made by conventional spinneret extrusion and drawing are not currently available smaller than about 6 micrometers in diameter, melt blowing, in which molten polymer is attenuated into fibers by a high velocity stream of gas and collected as a non-woven web is capable of making fibers on the order of a micrometer in diameter and has been essentially extended to the lower limit of fiber diameter with which coherent webs can be made. Webs with fiber diameters of about 1.5 to about 2 micrometers or less have been achieved, although very small diameter fibers may be difficult to collect as a continuous web, and may be less useful at normal working differential pressures.

The porous medium may be fashioned in a variety of ways to effectively separate or remove matter from the biological fluid passing through the medium. The porous medium may be any medium or combination of media which maintain leukocyte removal. The structure as provided by the present inventive porous medium may include single and/or multilayers. The porous medium is fibrous, and the fibers of such a medium may be microfibrous, woven, or unwoven.

The layers and/or fibers of such a porous medium may be compressed, bonded, fused, or otherwise fixed to one another, or they may simply be mechanically entwined. The porous medium may be hot compressed. The porous medium may be configured in any way deemed desirable, for example, as a web, matrix, flat sheet, depth filter, or in a corrugated or pleated structure. A pleated porous medium may have longitudinally extending pleats having peaks. The fiber diameter and/or void spaces may vary in a continuous or stepwise manner. Included within the scope of the present invention, particularly with respect to an extracorporeal circuit, is a pleated fibrous porous medium having at least two layers of porous medium.

The pores of the porous media provided by the present invention may have any pore diameter suited to the particular contemplated use, such as, for example, leukocyte depletion from circulating blood, buffy coat, packed red cells, platelet containing solutions, and plasma. The term,"pore diameter" as used herein is determined by the modified OSU F2 test described in detail in U.S. Patent 4,880,548. Generally, the pore diameter of the porous medium used in the invention may be in the range of from about 0.5 µm to about 50 µm. Typically, the pore diameter for leukocyte removal from packed red cells will be relatively smaller than the pore diameter of a porous medium used for leukocyte depletion from platelet rich plasma and for circulating blood in an extracorporeal circuit. A larger pore rating, on the order of about 6 µm or so, may be used while still retaining desirable efficiency with satisfactory surface modification of the porous medium. The optimization of the pore rating of the porous medium for a particular use will be well understood by those skilled in the art.

It is generally desirable to include the porous medium provided by the present invention in a housing compatible with the biological fluid. A filter assembly comprises a housing, having an inlet and an outlet, and a porous medium disposed in the housing for decreasing the leukocyte content and/or removing other undesirable matter from a biological fluid.

A filter assembly for use in an extracorporeal circuit comprises a housing, having an inlet and an outlet, and a fibrous medium disposed in the housing for decreasing the leukocyte content and removing other deleterious matter from a leukocyte-containing fluid. The filter assembly may also comprise a degassing mechanism cooperatively arranged with the housing for removing gaseous emboli from the liquid.

The filter assembly may be configured in a variety of ways. For example, a filter assembly in accordance with an extracorporeal circuit may include a hollow porous medium which may have a cylindrical shape and may be disposed in the housing to filter liquid flowing laterally or radially through the medium. For example, to filter liquid flowing inside/out through the porous medium, the inlet and outlet of the filter assembly would be arranged to respectively communicate with the interior and exterior of the hollow porous medium.

In the illustrated embodiment, the filter assembly is arranged to filter liquid flowing outside/in through the porous medium. This arrangement is generally used in an extracorporeal embodiment because it provides a porous medium with a large surface area in a compact housing.

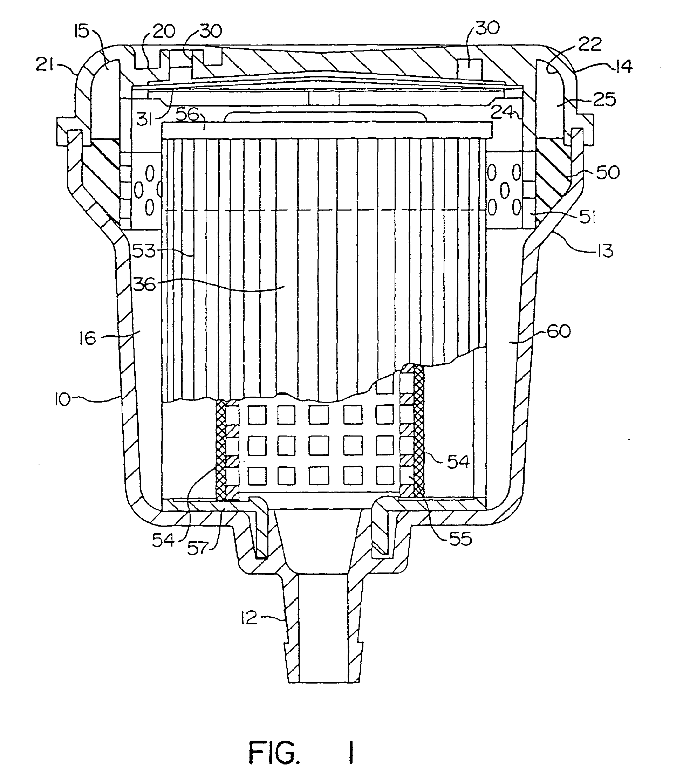

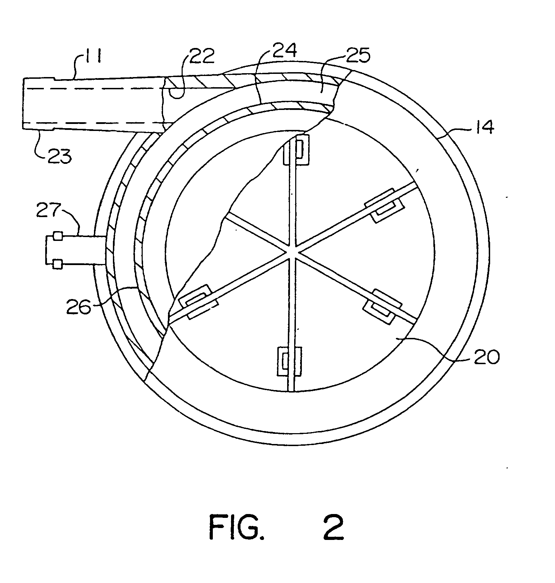

Any housing of suitable shape to provide an inlet and an outlet for liquid and a space for a porous medium disposed between the inlet and outlet can be employed. Housings can be designed to accept a variety of shapes of filter assemblies. For example, a square or octagon shaped housing and other possible forms designed to accommodate a similarly shaped porous medium would in principle all be functional, provided that adequate flow area is provided by the porous medium. These shapes are within the scope of the claimed invention. A filter assembly for use in an extracorporeal embodiment comprises a generally cylindrical housing 10 having an inlet 11 and an outlet 12, as shown in Figures 1 and 2.

Any housing of suitable configuration to reliably contain the liquid and define a liquid flow path through the porous medium can be employed. A filter assembly for use in an extracorporeal circuit comprises a housing 10 which generally includes two parts, a body 13 and a cover 14, and defines upper and lower chambers 15, 16. The cover 14 has a shallow, generally cylindrical configuration and includes a generally flat top wall 20 and a downturned, generally cylindrical side wall 21.

In the illustrated embodiment, the cover 14 includes the inlet 11, as shown in Figure 2. The inlet 11 may be variously configured. For example, the inlet 11 may comprise a nipple 23 which defines an inlet passage 22 and may be molded integrally with the cover 14. In the illustrated embodiment, the inlet 11 is configured to receive the end of a tube (not shown). In a typical embodiment, the inlet passage 22 is horizontal and opens through the side wall of the cover 14 in a direction tangential to the side wall.

The cover 14 may also be provided with an accessory port 27 and an annular baffle 24. The accessory port 27 may be used to provide pressure measurements or samples of the liquid being filtered. When it is not in use, the accessory port 27 may be capped. The annular baffle 24 is preferably concentric with and spaced inwardly from the side wall 21. The baffle 24 may be formed integrally with the cover 14, extending downwardly from the top wall 20, and may be generally coextensive with the side wall 21, forming a circular channel portion 25 in the upper chamber 15. An opening 26 in the baffle 24 allows the circular channel 25 to communicate with a vent in the cover 14.

The vent allows gas to escape from the housing and may be configured in a variety of ways. For example, it may comprise a nipple with a manually operable valve. However, in a typical embodiment, the vent comprises one or more holes 30 spaced around the top wall 20 of the cover 14. A porous, liquophobic membrane 31 may cover the holes 30 allowing gas but not liquid to escape from the housing. In an embodiment, the liquophobic membrane may be attached to the underside of the top wall 20 of the cover 14 to allow a relatively free flow of gas from the housing. The liquophobic membrane may be variously configured. For example, it may comprise a polytetraflucroethylene membrane having an absolute pore rating of about 0.2 µ and a polypropylene backing as a support.

The housing may be fabricated from any sufficiently rigid, impervious material which is compatible with the biological fluid. For example, the housing may be fabricated from a metal, such as stainless steel, or from a polymer. In a typical embodiment, the housing is fabricated from a plastic material, such as polystyrene, polycarbonate, or polypropylene. In addition, all of the surfaces of the housing which contact the liquid are preferably liquophilic, i.e., readily wettable by the fluid. For example, with respect to an extracorporeal embodiment, the internal surfaces of the body 13 and the cover 14 may be treated to achieve a high degree of liquophilicity, e.g., by surface graft copolymerization of hydroxyl functional monomers or by subjecting the internal surfaces to gas plasma treatment, as noted above. These liquophilic internal surfaces then readily facilitate the release of gas bubbles during the preparation and priming operation. A method of reducing the adhesion of bubbles in medical equipment is disclosed in U.S. Patent 4,861,617.

The housing may also be constructed of materials which have been exposed to a gas plasma in much the same manner as the porous medium. Such a gas plasma treated housing could, therefore, exhibit some of the same improved characteristics as the porous medium provided by the present invention, e.g., minimizing the retention of platelets during the separation or removal of blood components.

The degassing element 50 may be fashioned from any material which causes small gas bubbles in the fluid to coalesce and separate from the fluid. In a typical embodiment, the degassing element is a porous structure such as a porous foam or sponge material, and may include a rigid porous member 51 which engages the pleated porous medium. In addition, the degassing element may be treated with an anti-foaming agent to aid in breaking down the film between bubbles, for example, a compound of silicone and silica, such as Medical Antifoam A, available from Dow Corning Mfg. Co.

With respect to an extracorporeal embodiment, the porous medium, which may comprise a fibrous structure made from any material compatible with the liquid, may also include structures such as end caps, edge seals, a cage, a core, or a wrap. The porous medium also may include a downstream screen, preferably about a 40 micron screen, suitable for removing debris greater than about 40 microns.

As shown in Figure 1, the illustrated embodiment has a hollow, generally cylindrical configuration and comprises a porous medium 36 including a pleated medium 53, a porous element or screen 54, a perforated core 55, an upper blind end cap 56, and a lower open end cap 57. The porous medium is preferably disposed within the lower chamber 16 in the housing 10 and is smaller in diameter than the side wall of the body 13 so that an annular space 60 is left between the side wall and the porous medium 36. The interior of the porous medium communicates with the centrally located outlet 12.

The porous element or screen 54, which preferably has a pore size no greater than about 40 microns, is disposed coaxially adjacent to the downstream surface of the pleated medium, e.g., around the interior of the pleated medium. The porous element 54 may be fashioned from any compatible porous membrane or woven or non-woven material, including a mesh or a screen. The porous element 54 serves principally as a final filter to remove, for example, any aggregates which escape the pleated medium or form at the downstream portion of the pleated medium.

The perforated core 55 is disposed within and adjacent to the interior of the porous element 54 and serves principally to support the pleated medium 53 and the porous element 54 against the differential pressure across the porous medium 36. Consequently, the perforated core 55 may be fashioned from any suitably rigid material including a metal such as stainless steel or a rigid polymer such as polyolefin, polyester, or polyacrylate.

The end caps 56, 57 serve to direct the liquid radially outside/in through the porous medium 36. Both end caps 56, 57 may be fashioned from any suitably impervious material, such as a metallic or polymeric material, preferably a polymer such as polypropylene, which is compatible with the fluid to be filtered; the end caps are fixed to the respective ends of the pleated medium, the porous element 54, and the perforated core 55. Alternatively, the lower ends of the pleated medium, the porous element, and the perforated core may be fixed directly to the bottom wall of the body, eliminating the need for a lower end cap. The end caps 56 and 57 may be secured to the ends of the porous medium by any suitable means, including a bonding agent such as an adhesive or a potting compound. Alternatively, the end caps 56 and 57 may be melt-bonded to the ends of the porous medium or joined by means of spin bonding or sonic welding. The ends of the hollow core 55 may be secured to the two end caps 56 and 57 by similar means.

A blind end cap 56 and an open end cap 57 may be fitted over the two ends of the porous medium to direct fluid through the porous medium. Alternatively, both end caps can be open or can include connectors to link a stack of porous media.

Alternatively, the porous medium for use in an extracorporeal embodiment may be designed for inside/out flow. The porous element may then be disposed around the exterior of the fibrous porous medium, the upper end cap may be an open end cap, and the lower end cap may be a blind end cap. The core may be omitted but a cage disposed coaxially around the porous element to support the fibrous porous medium and the porous element against the pressure drop may be added. Of course, the housing would be rearranged to permit the inlet to communicate with the interior of the porous medium and the outlet to communicate with the exterior of the porous medium.

In the illustrated embodiment, the annular thickness of the pleated structure of a fibrous porous medium is preferably in the range from (0.1 to about 3 inches) .25 cm to 7.62 cm, more preferably in the range from about (0.2 to about 0.8 inch) .51 to 2.0 cm, and most preferably in the range from about (0.3 to about 0.6 inch) .76 to 1.52 cm. The outer diameter of the fibrous medium is preferably less than about (8.5 inches) 21.6 cm, more preferably less than about (3 inches) 7.62 cm. The height of the pleated structure is preferably up to about (5.5 inches) 14 cm, more preferably about (2.5 inches) 6.4 cm. The hold up volume of the filter assembly is preferably in the range of about 70 cc to about 400 cc or even greater, more preferably about 100 cc to about 250 cc.

In a preferred embodiment for use in an extracorporeal circuit, the fibrous porous medium may be formed into a sheet; multiple sheets may then be layered and pleated using conventional pleating equipment. The pleated structure typically includes about 4 - 16 pleats per 2.54 cm, preferably about 10 pleats per 2.54 cm. The height of the pleats are typically about (.2 to about .6 inch) 0.51 to 1.52 cm., preferably about (.4 inch) 1.02 cm.

The present invention also provides a method for removing leukocytes from biological fluids such as blood. The method comprises depleting the content of leukocytes from biological fluids by passing the fluid through a fibrous porous medium that has been treated with gas plasma generated from a mixture of gases including argon.

The flow rate of liquid passing through a fibrous porous medium or a filter assembly provided by the present invention can vary according to the particular use. Additionally, particularly with respect to an extracorporeal circuit, the flow rate can vary for any given patient. However, in either case, the flow rate should be maintained at a level which does not harm or destroy erythrocytes or platelets in the liquid. With respect to passing the biological fluid extracorporeally, embodiments of the invention may filter as little as about 25 milliliters per minute or may have the capacity to filter up to about 6 liters per minute, without clogging (i.e., without increasing the pressure across the porous medium to above about 21.3 x 10³kg/m³ (15 psi)).

Typically, in an extracorporeal embodiment of the invention, a biological fluid enters a filter assembly provided by the present invention through inlet passage 22 and into the circular channel 25 in upper chamber 15 where a generally circular liquid flow pattern is maintained by annular baffle 24 and the side wall 21 of the cover 14. This flow pattern produces a centrifugal force which causes at least some of the gas bubbles in the fluid, including any gross gas bubbles, to separate from the fluid and move inwardly and through the opening 26 in the baffle 24 into the central portion of the upper chamber 15. The gas in the fluid is then vented from the filter assembly through the holes 30 in the cover 14. Typically, the gas passes through a liquophobic membrane 31, which covers the holes 30 and prevents the fluid from escaping from the housing 10.

Typically, the fluid in channel 25 then passes through the annular sponge 50 and the perforated ring 51 to the space 60 in the lower chamber. The degassing element 50 brakes the rotational flow of the fluid and dissipates the centrifugal forces which might otherwise tend to force gas bubbles toward the porous medium 36. Also, as the fluid passes through the degassing element 50, any smaller gas bubbles remaining in the fluid coalesce into larger bubbles which, as the fluid flows through the perforations in the perforated ring 51, rise to the central portion of the upper chamber 15 and are vented from the filter assembly as noted above. Thus, the fluid which flows into the space 60 is substantially degassed.

In the embodiment provided by the invention characterized as "outside/in," the degassed fluid then passes from space 60 through porous medium 36 and into the interior of the medium. The filtered fluid then flows from the interior of the medium and exits from the housing 10 by passing through outlet 12.

While some of the extracorporeal devices described herein are principally directed to a filter assembly having a capacity of passing up to about 6 liters/minute, filter assemblies having a larger or smaller capacity can be made. Included within the scope of the invention is a filter assembly designated as a "low flow" size, which has a flow rate of about 3 liters/minute or less, has approximately one-third the fiber surface area and about one-half the capacity of the adult device.

An extracorporeal filter assembly as provided by the present invention has the capacity for up to about 10 hours of continuous removal of a clinically or therapeutically significant amount of leukocytes and other deleterious matter from a biological fluid. However, many of the uses for which these filter assemblies are suitable do not require 10 hours of filtration. For example, a cardiac bypass operation may only require 6-8 hours; cardioplegia may require only 2-4 minutes of filtration. Some therapeutic protocols performed under emergency conditions require only 10-20 seconds of filtration, or several periodic or repeated filtrations of about 10-20 seconds duration.

A gas plasma treated porous medium as provided by the present invention is capable of decreasing the leukocyte content of a biological fluid in any protocol involving treatment of the biological fluid. This generally means removing a therapeutically or clinically significant amount of leukocytes from a biological fluid. "Therapeutically or clinically significant amount" refers to an amount necessary to produce a beneficial effect on the patient or animal receiving the leukocyte depleted liquid. Such a beneficial effect may be, for example, lessening reperfusion injury. A therapeutically or clinically significant amount can vary depending on the intended use and/or from patient to patient. For example, a therapeutically or clinically significant amount can be greater for a cardiac bypass procedure than for cardioplegia. However, removal of a therapeutically or clinically significant amount can be and is routinely determined by a doctor or technician for treating a certain condition or disease as it pertains to the specific patient or animal, and as it pertains to the particular application.

A porous medium or filter assembly provided by the present invention may be used in any procedure, therapy, operation, or environment in which the removal of activated leukocytes and/or other undesirable matter is desirable or beneficial. Because leukocytes have the potential for becoming activated upon contact with almost anything ex-vivo, many applications exist for the use of the porous media and filter assemblies provided by the present invention in reducing the number of activated leukocytes. While the filter assembly provided by the present invention is particularly suited for treating reperfusion-induced injury and/or achieving leukocyte content equilibrium in an extracorporeal system, one skilled in the art will recognize other contexts in which removal of leukocytes and other deleterious matter in a liquid is desirable.

It should be understood that the specific embodiments described above are not intended to limit the invention, but, on the contrary, the intention is to cover all alternatives falling within the scope of the invention as defined in the claims.

## Claims

1. A leukocyte depletion medium (36) for removing leukocytes from a biological fluid comprising: a fibrous porous medium (36) which has been treated with a plasma generated from a mixture of gases, the mixture of gases including argon.

2. A medium (36) according to claim 1 comprising synthetic fibers.

3. A medium (36) according to any preceding claim wherein the mixture of gases includes at least one organic gas.

4. A medium (36) according to any preceding claim wherein the medium (36) has been treated without introducing a polymeric substance into the plasma.

5. A medium (36) according to any preceding claim wherein said porous medium (36) has been treated with a gas plasma at about 10 to about 3000 watts, about 1 kHz to about 100 Mhz, and at about 0.001 to about 100 torr for about 5 seconds to about 12 hours.

6. A method for the separation of leukocytes from a biological fluid comprising: passing the fluid through a leukocyte depletion medium (36) comprising a fibrous porous medium (36) which has been treated with a gas plasma generated from a mixture of gases including argon, and depleting leukocytes from the biological fluid.

7. A method according to claim 6 wherein the fibrous porous medium (36) comprises synthetic fibers.

8. A method according to claim 6 or claim 7 wherein the biological fluid includes platelets.

9. A method according to claim 8 wherein said leukocyte depletion medium (36) allows the passage of a majority of said platelets therethrough.

10. A method according to any one of claims 6-9 wherein the biological fluid includes red cells.

11. A method according to any one of claims 6-10 wherein passing the biological fluid through the leukocyte depletion medium (36) comprises passing the fluid through a porous medium (36) which has been treated with a gas plasma without introducing a polymeric substance into the plasma.

12. A method according to any one of claims 6-11 wherein the mixture of gases includes at least one organic gas.

13. A method for making a leukocyte depletion medium comprising: generating a plasma from a mixture of gases including argon; and treating a fibrous porous medium (36) with the plasma.

14. A method according to claim 13 wherein the porous medium (36) comprises synthetic fibers.

15. A method according to claim 13 or claim 14 wherein treating includes treating the porous medium with a gas plasma at about 10 to about 3000 watts, about 1 kHz to about 100 Mhz, and at about 0.001 to about 100 torr for about 5 seconds to about 12 hours.

## Patentansprüche

1. Leukozyten-Abreicherungsmedium (36) zum Entfernen von Leukozyten aus einem biologischen Fluid, umfassend: ein faserförmiges poröses Medium (36), welches mit einem aus einer Mischung von Gasen erzeugten Plasma behandelt worden ist, wobei die Mischung von Gasen Argon umfasst.

2. Medium (36) nach Anspruch 1, umfassend synthetische Fasern.

3. Medium (36) nach einem der vorangehenden Ansprüche, wobei die Mischung von Gasen mindestens ein organisches Gas umfasst.

4. Medium (36) nach einem der vorangehenden Ansprüche, wobei das Medium (36) behandelt worden ist, ohne einen polymeren Stoff in das Plasma einzuführen.

5. Medium (36) nach einem der vorangehenden Ansprüche, wobei das poröse Medium (36) mit einem Gasplasma bei ca. 10 bis ca. 3.000 Watt, ca. 1 kHz bis ca. 100 MHz und bei ca. 0,001 bis ca. 100 Torr für ca. 5 Sekunden bis ca. 12 Stunden behandelt worden ist.

6. Verfahren zum Abtrennen von Leukozyten aus einem biologischen Fluid, umfassend: Durchführen des Fluids durch ein Leukozyten-Abreicherungsmedium (36), umfassend ein faserförmiges poröses Medium (36), welches mit einem aus einer Mischung von Gasen, umfassend Argon, erzeugten Gasplasma behandelt worden ist; und Abreichern von Leukozyten aus dem biologischen Fluid.

7. Verfahren nach Anspruch 6, wobei das faserförmige poröse Medium (36) synthetische Fasern umfasst.

8. Verfahren nach Anspruch 6 oder Anspruch 7, wobei das biologische Fluid Thrombozyten umfasst.

9. Verfahren nach Anspruch 8, wobei das Leukozyten-Abreicherungsmedium (36) den Durchtritt einer Mehrheit der Thrombozyten erlaubt.

10. Verfahren nach einem der Ansprüche 6 bis 9, wobei das biologische Fluid rote Blutkörperchen umfasst.

11. Verfahren nach einem der Ansprüche 6 bis 10, wobei das Durchführen des biologischen Fluids durch das Leukozyten-Abreicherungsmedium (36) das Durchführen des Fluids durch ein poröses Medium (36) umfasst, welches mit einem Gasplasma behandelt worden ist, ohne einen polymeren Stoff in das Plasma einzuführen.

12. Verfahren nach einem der Ansprüche 6 bis 11, wobei die Mischung von Gasen mindesten ein organisches Gas umfasst.

13. Verfahren zur Herstellung eines Leukozyten-Abreicherungsmediums, umfassend: Erzeugen eines Plasmas aus einer Mischung von Gasen, umfassend Argon; und Behandeln eines faserförmigen porösen Mediums (36) mit dem Plasma.

14. Verfahren nach Anspruch 13, wobei das poröse Medium (36) synthetische Fasern umfasst.

15. Verfahren nach Anspruch 13 oder Anspruch 14, wobei das Behandeln umfasst: Behandeln des porösen Mediums mit einem Gasplasma bei ca. 10 bis ca. 3.000 Watt, ca. 1 kHz bis ca. 100 MHz und bei ca. 0,001 bis ca. 100 Torr für ca. 5 Sekunden bis ca. 12 Stunden.

## Revendications

1. Milieu d'appauvrissement en leucocytes (36) pour éliminer les leucocytes dans un fluide biologique, comprenant : un milieu poreux fibreux (36) qui a été traité avec un plasma généré à partir d'un mélange de gaz, le mélange de gaz comprenant de l'argon.

2. Milieu (36) selon la revendication 1, comprenant des fibres synthétiques.

3. Milieu (36) selon l'une quelconque des revendications précédentes, dans lequel le mélange de gaz comprend au moins un gaz organique.

4. Milieu (36) selon l'une quelconque des revendications précédentes, dans lequel le milieu (36) a été traité sans introduction d'une substance polymère dans le plasma.

5. Milieu (36) selon l'une quelconque des revendications précédentes, dans lequel ledit milieu poreux (36) a été traité avec un plasma gazeux à environ 10 à environ 3000 watts, environ 1 kHz à environ 100 MHz, et sous environ 0,001 à environ 100 Torr, pendant environ 5 secondes à environ 12 heures.

6. Procédé pour la séparation de leucocytes à partir d'un fluide biologique, comprenant : le passage du fluide à travers un milieu d'appauvrissement en leucocytes (36) comprenant un milieu poreux fibreux (36) qui a été traité avec un plasma gazeux généré à partir d'un mélange de gaz contenant de l'argon, et l'appauvrissement en leucocytes du fluide biologique.

7. Procédé selon la revendication 6, dans lequel le milieu poreux fibreux (36) comprend des fibres synthétiques.

8. Procédé selon la revendication 6 ou la revendication 7, dans lequel le fluide biologique comprend des plaquettes.

9. Procédé selon la revendication 8, dans lequel ledit milieu d'appauvrissement en leucocytes (36) permet le passage d'une majorité desdites plaquettes à travers celui-ci.

10. Procédé selon l'une quelconque des revendications 6 à 9, dans lequel le fluide biologique comprend des globules rouges.

11. Procédé selon l'une quelconque des revendications 6 à 10, dans lequel le passage du fluide biologique à travers le milieu d'appauvrissement en leucocytes (36) comprend le passage du fluide à travers un milieu poreux (36) qui a été traité avec un plasma gazeux sans introduction d'une substance polymère dans le plasma.

12. Procédé selon l'une quelconque des revendications 6 à 11, dans lequel le mélange de gaz contient au moins un gaz organique.

13. Procédé pour produire un milieu d'appauvrissement en leucocytes, comprenant : la génération d'un plasma à partir d'un mélange de gaz contenant de l'argon ; et le traitement d'un milieu poreux fibreux (36) avec le plasma.

14. Procédé selon la revendication 13, dans lequel le milieu poreux (36) comprend des fibres synthétiques.

15. Procédé selon la revendication 13 ou la revendication 14, dans lequel le traitement comprend le traitement du milieu poreux avec un plasma gazeux à environ 10 à environ 3000 watts, environ 1 kHz à environ 100 MHz, et sous environ 0,001 à environ 100 Torr, pendant environ 5 secondes à environ 12 heures.
